# FASCICULE DE BREVET EUROPEEN

(11) **EP 1 523 321 B1**
(45) Date de publication et mention de la délivrance du brevet: **02.12.2009**
(21) Numéro de dépôt: 03755604.0
(22) Date de dépôt: 15.07.2003
(51) Int. Cl.: A61K 36/16, A61P 3/04

(54) **UTILISATION D'EXTRAITS DE GINKGO BILOBA POUR FAVORISER LA MASSE MUSCULAIRE AU DETRIMENT DE LA MASSE GRAISSEUSE**
VERWENDUNG VON GINKGO BILOBA EXTRAKTEN ZUR FÖRDERUNG DER MUSKELMASSE ZUM NACHTEIL DER FETTMASSE
USE OF GINKGO BILOBA EXTRACTS IN ORDER TO PROMOTE MUSCLE MASS TO THE DETRIMENT OF FATTY MASS

(30) Priorité: 16.07.2002 FR 0208941
(43) Date de publication de la demande: 20.04.2005
(73) Titulaire: IPSEN PHARMA, 92100 Boulogne-Billancourt (FR)
(72) Inventeur: CHRISTEN, Yves, F-75116 Paris (FR)
(74) Mandataire: Bourgouin, André
(86) Numéro de dépôt international: PCT/FR2003/002230
(87) Numéro de publication internationale: WO 2004/014405

(56) Documents cités:
- EP-A- 0 427 026
- EP-A- 0 493 151
- EP-A- 1 093 817
- EP-A1- 0 431 535
- EP-B1- 1 098 657
- US-A- 5 389 370
- US-A- 6 143 725
- DATABASE WPI Section Ch, Week 200014 Derwent Publications Ltd., London, GB; Class B04, AN 2000-148148 XP002236988 & CN 1 235 784 A (BAO D), 24 novembre 1999 (1999-11-24)
- JOURNAL ARTICLE: 'GINKGO BILOBA COMMON NAME: GINKGO, MAIDENHAIR TREE' ALTERNATIVE MEDICINE REVIEW : A JOURNAL OF CLINICAL THERAPEUTIC. vol. 3, no. 1, 01 Février 1998, THORNE RESEARCH INC., SANDPOINT, US, pages 54 - 57, XP007900616
- DRIEU K.: 'PREPARATION ET DEFINITION DE L'EXTRAIT DE GINKGO BILOBA//PREPARATION AND DEFINITION OF GINKGO BILOBA EXTRACT' LA PRESSE MEDICALE no. 31, 25 Septembre 1986, PARIS, FRANCE, pages 1455 - 1457, XP009041461
- PROIETTO J. ET AL: 'Novel anti-obesity drugs' EXP. OPIN. INVETS. DRUGS vol. 9, no. 6, Juin 2000, pages 1317 - 1326, XP001004696
- CLAPHAM J.C. ET AL: 'Anti-obesity drugs: a critical review of current therapies and future opportunities' PHARMACOLOGY AND THERAPEUTICS vol. 89, 2001, pages 81 - 121, XP001120530

## Description

La présente demande de brevet concerne l'utilisation d'extraits de *Ginkgo biloba* pour préparer un médicament destiné à favoriser la masse musculaire au détriment de la masse graisseuse.

L'obésité est un véritable problème de santé publique. Certains médicaments permettent bien de provoquer une perte ou un gain de poids, mais le traitement affaiblit généralement les patients chez qui la perte ou le gain de poids se fait souvent au détriment du rapport de la masse musculaire sur la masse graisseuse.

La demanderesse a à présent découvert que l'administration d'extraits de *Ginkgo biloba* chez des sujets essayant de perdre ou gagner du poids a l'effet bénéfique de favoriser la masse musculaire au détriment de la masse graisseuse. En outre, la demanderesse a pu constater que ladite administration provoque une perte de poids chez le sujet en surpoids auquel il est administré.

D'une façon générale, l'utilisation d'extraits de *Ginkgo biloba* pour préparer un médicament destiné à traiter les problèmes de poids, ceci aussi bien chez les personnes cherchant à gagner du poids qu'à en perdre 4 est décrite.

L'invention concerne en particulier l'utilisation d'extraits de *Ginkgo biloba* pour préparer un médicament destiné à perdre du poids. La perte de poids chez le sujet traité sera d'au moins 4 ou 5%, et plus préférentiellement d'au moins 6, 8 ou 10% de sa masse corporelle totale.

Selon une variante particulière de l'invention, les extraits de *Ginkgo biloba* seront utilisés pour préparer un médicament destiné à favoriser la masse musculaire au détriment de la masse graisseuse chez des patients cherchant à perdre ou à gagner du poids. Ces patients seront éventuellement soumis à un régime et/ou à un autre traitement médical.

En d'autres termes, lorsque, le patient cherchant à gagner ou perdre du poids est traité avec un extrait de *Ginkgo biloba* de façon concomitante à son régime ou à son autre traitement médical, le rapport R égal à sa masse musculaire Mₘ divisée par sa masse corporelle totale Mₜ a tendance à rester stable ou, dans la plupart des cas, à augmenter. De préférence, l'augmentation ainsi obtenue après une période de traitement d'un mois au moins avec un extrait de *Ginkgo biloba* sera supérieure ou égale à 5%, et plus préférentiellement supérieure ou égale à 6 voire 8 ou 10%.

Les extraits de *Ginkgo biloba* utilisables seront tels qu'ils comportent au moins des flavoneglycosides et / ou un ou des ginkgolides. De préférence, les flavoneglycosides et / ou le ou les ginkgolides seront présents au moins à hauteur de 25% en poids, plus préférentiellement au moins à hauteur de 30% en poids et encore plus préférentiellement au moins à hauteur de 50% en poids dans l'extrait de *Ginkgo biloba* utilisé pour préparer le médicament selon l'invention. Par ailleurs, la proportion de composés de type alkylphénols dans l'extrait de *Ginkgo biloba* utilisé selon l'invention sera de préférence inférieure à 10 ppm, plus préférentiellement inférieure à 5 ppm et encore plus préférentiellement inférieure à 1 ppm. Le cas échéant, le ou les ginkgolides pourront être remplacés par leurs homologues acétylés, leurs homologues alkoxylés ou leurs homologues glycosylés (comme par exemple les composés de formule générale **(I)** décrite ci-après).

De préférence, l'extrait de *Ginkgo biloba* utilisé pour préparer un médicament selon l'invention sera enrichi en flavoneglycosides et / ou en ginkgolides. Il pourra par exemple s'agir d'un extrait de type EGb 761^{®}. Selon une autre variante de l'invention, l'extrait de *Ginkgo biloba* utilisé pour préparer un médicament selon l'invention sera tout extrait de *Ginkgo biloba* contenant des flavoneglycosides, des ginkgolides et du bilobalide, par exemple un extrait de type CP 401.

Par extrait de type EGb 761^{®}, on entend un extrait de composition sensiblement identique à celle de l'extrait standardisé EGb 761^{®} tel qu'il a été défini notamment dans l'article suivant: K. Drieu, La presse médicale, 31, 25 septembre 1986, supplément consacré à l'extrait de *Ginkgo biloba* (EGb 761^{®}), 1455-1457 ; ou dans les brevets européens EP 431 535 et EP 431 536 ; par extrait de type EGb 761^{®}, on entend donc notamment les extraits de *Ginkgo biloba* comprenant de 20 à 30 % de flavoneglycosides, de 2,5 à 4,5 % au total de ginkgolides A, B, C et J, de 2 à 4 % de bilobalide, moins de 10 % de proanthocyanidines et moins de 10 ppm (de préférence moins de 5 ppm et encore plus préférentiellement moins de 1 ppm) de composés de type alkylphénols, de préférence les extraits de *Ginkgo biloba* comprenant de 22 à 36 % de flavoneglycosides, de 2,5 à 3,5 % au total de ginkgolides A, B, C et J, de 2,5 à 3,5 % de bilobalide, moins de 8 % de proanthocyanidines et moins de 10 ppm (de préférence moins de 5 ppm et encore plus préférentiellement moins de 1 ppm) de composés de type alkylphénols, et en particulier les extraits de Ginkgo biloba comprenant environ 24 % de flavoneglycosides, 3,1 % au total de ginkgolides A, B, C et J, 2,9 % de bilobalide, 6,5 % de proanthocyanidines et moins de 1 ppm de composés de type alkylphénols.

Par extrait de type CP 401, on entend des extraits tels que ceux qui sont présentés dans le brevet US 5,389,370, notamment les extraits de *Ginkgo biloba* comprenant de 5,5 à 8 % au total de ginkgolides A, B, C et J, de 40 à 60 % de flavoneglycosides et de 5 à 7 % de bilobalide, de préférence les extraits de *Ginkgo biloba* comprenant de 6,5 à 7,5 % au total de ginkgolides A, B, C et J, de 45 à 55 % de flavoneglycosides et de 5,5 à 6,5 % de bilobalide et tout particulièrement les extraits comprenant environ 7 % au total de ginkgolides A, B, C et J, 50 % de flavoneglycosides et 6 % de bilobalide.

Par extension, seront également assimilés aux extraits de type EGb 761^{®} ou CP 401 les extraits de type EGb 761^{®} ou CP 401 dont les ginkgolides auront été remplacés par leurs homologues de formule générale **(I)** décrite plus loin.

Au moins une partie du ou des ginkgolides pourra être remplacée par les composés de formule générale **(I)** 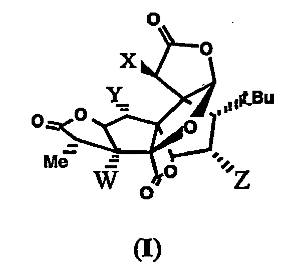 dans laquelle W, X, Y et Z représentent indépendamment les radicaux H, OH, alkoxy linéaire ou ramifié ou O-Gₛ, Gₛ-OH représentant un mono- ou un disaccharide, ou un de leurs dérivés ou analogues,
étant entendu que l'un au moins de W, X, Y ou Z représente un radical O-Gₛ.

De préférence, les composés de formule générale (I) décrits précédemment seront tels que X représente un radical OH ou O-Gₛ, Gₛ-OH représentant un mono- ou un disaccharide, ou un de leurs dérivés ou analogues, et :
- ou bien W représente un radical OH ou O-Gₛ, Y représente H et Z représente H ;
- ou bien W représente un radical OH ou O-Gs, Y représente un radical OH ou O-G_{S} et Z représente H ;
- ou bien W représente un radical OH ou O-Gₛ, Y représente un radical OH ou O-Gₛ et Z représente un radical OH ou O-G_{S} ;
- ou bien W représente un radical OH ou O-Gₛ, Y représente H et Z représente un radical OH ou O-G_{S} ;
- ou bien W représente H, Y représente un radical OH ou O-G_{S} et Z représente un radical OH ou O-Gₛ ;
- ou bien W représente un radical OH ou O-G_{S}, Y représente un radical alkoxy linéaire ou ramifié et Z représente H.

Pour la préparation des composés de formule générale (I), l'homme du métier pourra se référer à la demande de brevet PCT WO 98/52959 ou au brevet US 6,143,725.

Selon un aspect de l'invention, le patient aura du poids à perdre. Dans ce cas là, l'extrait de *Ginkgo biloba* pourra par exemple lui être administré en association avec un médicament contenant de l'orlistat (par exemple le Xenical^{®}), de la sibutramine ou un de ses sels pharmaceutiquement acceptables (par exemple le Sibutral^{®}), des extraits hydroalcooliques de thé vert (par exemple l'Exolise^{®} ou le Mincifit^{®}) ou du thé vert (par exemple la Camiline^{®}), ou avec tout autre médicament destiné à provoquer une perte de poids. De préférence l'extrait de *Ginkgo biloba* sera administré en association avec un médicament contenant de l'orlistat (par exemple le Xenical^{®}), de la sibutramine ou un de ses sels pharmaceutiquement acceptables (par exemple le Sibutral^{®}). Toutefois, lorsque le poids à perdre sera relativement faible (par exemple au inférieur ou égal à 5%, voire inférieur ou égal à 10% de la masse corporelle totale), le patient pourra simplement recevoir de l'extrait de *Ginkgo biloba* en sus du régime alimentaire qui lui est prescrit.

Il est aussi décrit un produit comprenant au moins un extrait de *Ginkgo biloba* tel que décrit précédemment en association avec au moins un composé choisi parmi l'orlistat, la sibutramine ou un de ses sels pharmaceutiquement acceptables, des extraits hydroalcooliques de thé vert ou du thé vert pour une utilisation simultanée, séparée ou étalée dans le temps dans le traitement d'une surcharge pondérale.

Par utilisation thérapeutique simultanée, on entend dans la présente demande une administration de plusieurs principes actifs par la même voie et au même moment. Par utilisation séparée, on entend notamment dans la présente demande une administration de plusieurs principes actifs sensiblement au même moment par des voies différentes.

Par utilisation thérapeutique étalée dans le temps, on entend dans la présente demande une administration de plusieurs principes actifs à des moments différents et notamment un mode d'administration selon lequel l'ensemble de l'administration de l'un des principes actifs est effectué avant que l'administration de l'autre ou des autres ne commence. On peut ainsi administrer l'un des principes actifs pendant plusieurs mois avant d'administrer l'autre ou les autres principes actifs. Il n'y a pas de traitement simultané dans ce cas.

La composition pourra être utilisée lorsque le patient aura du poids à gagner. Dans ce cas, l'extrait de *Ginkgo biloba* pourra lui être administré en association avec un médicament comme le fenugrec (par exemple le Fénugrène^{®}) ou tout autre médicament destiné à provoquer un gain de poids. Toutefois, lorsque le poids à gagner sera relativement faible (par exemple au inférieur ou égal à 5%, voire inférieur ou égal à 10% de la masse corporelle totale), le patient pourra simplement recevoir de l'extrait de *Ginkgo biloba* en sus du régime alimentaire qui lui est prescrit.

Il est aussi décrit un produit comprenant au moins un extrait de *Ginkgo biloba* tel que décrit précédemment en association avec du fenugrec pour une utilisation simultanée, séparée ou étalée dans le temps dans le traitement d'un déficit pondéral.

Le patient ayant du poids à perdre ou à gagner pourra être un animal ou un être humain. Parmi les animaux pour lesquels, la composition pourra être utilisée, on peut notamment citer les chiens, les chats, les bovins, les ovins, les volailles (poules, dindes, canards, etc.) ou les chevaux. De préférence, le patient sera un être humain.

Les compositions pharmaceutiques comprenant un extrait de *Ginkgo biloba* peuvent être sous forme de solides, par exemple des poudres, des granules, des comprimés, des gélules, des liposomes, des suppositoires ou des timbres applicateurs (patchs). Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques comprenant un extrait de *Ginkgo biloba* peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament comprenant ces compositions pourra se faire par voie topique, orale, parentérale, par injection (intramusculaire, sous-cutanée, intraveineuse, etc.), etc.

La dose d'administration journalière d'extrait de *Ginkgo biloba* envisagée est comprise entre 0,1 mg à 10 g suivant la concentration de l'extrait en principes actifs et la gravité des problèmes de poids du sujet à traiter. Il en sera en définitive décidé par le médecin ou le vétérinaire traitant.

Le terme "environ" fait référence à un intervalle autour de la valeur considérée. Tel qu'utilisé dans la présente demande, "environ X" signifie un intervalle de X moins 10% de X à X plus 10% de X, et de préférence un intervalle de X moins 5% de X à X plus 5% de X.

A moins qu'ils ne soient définis d'une autre manière, tous les termes techniques et scientifiques utilisés ici ont la même signification que celle couramment comprise par un spécialiste ordinaire du domaine auquel appartient cette invention.

Afin de montrer l'intérêt de l'utilisation d'extraits de *Ginkgo biloba* tels que décrits précédemment dans le traitement des problèmes de poids, le test exposé ci-après peut être effectué. D'autres tests visant par exemple à déterminer la composition corporelle et notamment le rapport entre masse graisseuse et masse non graisseuse (cf. Chumlea et coll., *Nutrition, Health & Aging,* **1(1)**, 7-12) peuvent également être réalisés afin de parvenir au même résultat

### Partie pharmacologique

### Mesure comparative de l'évolution du poids du corps chez des rats âgés traités ou non par de l'EGb 761 :

Les rats de laboratoire âgés ont, du fait de leur captivité, une tendance naturelle à prendre du poids. Deux groupes de rats Wistar âgés (22 mois) sont créés, l'un étant constitué de 11 rats qui reçoivent une eau de boisson normale pendant 5 semaines et l'autre étant constitué de 12 rats qui reçoivent une eau de boisson contenant 75 mg par kg d'extrait de *Ginkgo biloba* standardisé EGb 761^{®}. Les rats sont pesés avant le début et après la fin du traitement.

### Résultats obtenus :

i) Les mesures du poids des rats fournissent les résultats suivants :

| | Rats âgés non traités | Rats âgés traités |
|---|---|---|
| Poids moyen à 22 mois (g) | 605,64 | 594,10 |
| Poids moyen à 22 mois et 5 semaines (g) | 619,33 | 570,11 |

En d'autres termes, en l'espace de quelques semaines, les rats non traités ont gagné 2,2% de masse corporelle tandis que les rats traités ont *perdu* 4% de leur masse corporelle.
ii) Par ailleurs, l'on observe surtout que le rapport du poids des muscles par rapport au poids total du corps est de 0,53 chez les rats traités contre 0,42 pour les rats non traités.

Par conséquent, on voit qu'un traitement par l'extrait de *Ginkgo biloba* standardisé EGb 761^{®} permet bien, d'une part, de faire perdre du poids aux rats ayant tendance à devenir obèses, et, d'autre part, de favoriser leur masse musculaire au détriment de leur masse graisseuse.

## Revendications

1. Utilisation d'un extrait de *Ginkgo biloba* comprenant
20 à 30 % de flavoneglycosides,
2,5 à 1,5 % au total de ginkgolides A, B, C et J,
2 à 4 % de bilobalide,
moins de 10 % de proanthocyanidines et moins de 10 ppm de composés de type alkylphénols,
pour la préparation d'un médicament destiné à traiter l'obésité en favorisant la masse musculaire au détriment de la masse graisseuse, et **caractérisé en ce que** l'extrait est administré seul.

2. Utilisation selon la revendication 1, **caractérisé en ce que** l'extrait de *Ginkgo biloba* comprend :
24 % le fluvoneglycosides.
3,1 % au total de ginkgolides A, B, C et J,
2,9 % de bilobalide,
6,5 % de proanthocyanidines et
moins de 1 ppm de composés de type alkylphénols.

## Claims

1. Use of a *Ginkgo biloba* extract comprising
from 20 to 30 % of flavoneglycosides,
from 2.5 to 4.5 % in total of ginkgolides A, B, C and J,
from 2 to 4 % of bilobalide,
less than 10 % of proanthocyanidines and less than 10 ppm of compounds of alkylphenol type.
for the preparation of a un medicament intended to treat obesity promoting muscle mass to the detriment of fatty mass, and **characterized in that** the extract is administered alone.

2. Use according to claim 1, **characterized in that** the *Ginkgo biloba* extract comprises :
24 % of flavoneglycosides,
3.1 % in total of ginkgolides A, B, C and J,
2.9% of bilobalide,
6.5 % of proanthocyanidines and
less than 1 ppm of compounds of alkylphenol type.

## Patentansprüche

1. Verwendung eines Extrakts von *Ginkgo biloba,* umfassend
20 bis 30 % Flavonglycoside,
insgesamt 2,5 bis 4,5 % Ginkgolide A, B, C und J,
2 bis 4 % Bilobalid,
weniger als 10 % Proanthocyanidine und weniger
als 10 ppm Verbindungen des Typs Alkylphenole, für die Herstellung eines Medikaments, das dazu bestimmt ist, Adipositas zu behandeln, indem es die Muskelmasse auf Kosten der Fettmasse fördert, und **dadurch gekennzeichnet, dass** der Extrakt allein verabreicht wird.

2. Verwendung gemäß Anspruch 1, **dadurch gekennzeichnet, dass** der Extrakt von *Ginkgo biloba* umfasst:
24 % Flavonglycoside,
insgesamt 3,1 % Ginkgolide A, B, C und J,
2,9 % Bilobalid,
6,5 % Proanthocyanidine und
weniger als 1 ppm Verbindungen des Typs Alkylphenole.
